Europäisches Patentamt

European Patent Office

Office européen des brevets

(11) Veröffentlichungsnummer: **0 312 494**

**A2**

(12) # EUROPÄISCHE PATENTANMELDUNG

(21) Anmeldenummer: **88810676.2**

(22) Anmeldetag: **30.09.88**

(51) Int. Cl.⁴: **A 61 L 27/00**
**A 61 L 31/00**

(30) Priorität: **16.10.87 CH 4081/87**

(43) Veröffentlichungstag der Anmeldung:
**19.04.89 Patentblatt 89/16**

(84) Benannte Vertragsstaaten:
**AT BE CH DE ES FR GB GR IT LI LU NL SE**

(71) Anmelder: **INSTITUT STRAUMANN AG**
**CH-4437 Waldenburg (CH)**

(72) Erfinder: **Steinemann, Samuel G., Prof. Dr.**
**Chemin des Codoz 14**
**CH-1025 St. Sulpice (CH)**

(74) Vertreter: **Eder, Carl E.**
**Patentanwaltsbüro EDER AG Münchensteinerstrasse 2**
**CH-4052 Basel (CH)**

(54) **Alloplastisches Material für die Bildung eines künstlichen und/oder Verstärkung eines natürlichen Weichgewebeteils.**

(57) Das zur Bildung eines künstlichen Weichgewebeteils und/oder zur Verstärkung eines natürlichen Weichgewebeteils, wie eines Bandes oder einer Sehne, dienende alloplastische Material (1) weist Bündel (3) von lose zusammengehaltenen, metallischen Fasern (5) auf. Die Fasern (5) bestehen vorzugsweise aus einer Legierung auf Titanbasis, die mindestens eines der Metalle Niob, Tantal, Zirkon, Chrom, Molybdän, Eisen und Aluminium enthält, und können mit einem Überzug aus einer in einem menschlichen oder tierischen Körper resorbierbaren, organischen Substanz versehen sein. An den Oberflächen von aus einer solchen Legierung bestehenden Fasern entstehen Oxydfilme, die das sich darunter befindende Metall gegen die im menschlichen oder tierischen Körper stattfindenden, chemischen Einwirkungen schützen. Ferner sind die als Legierungsbestandteile in den Fasern vorhandenen Metalle nicht toxisch und ermöglichen eine gute Bindung mit natürlichem Gewebe. Die Dicke der Fasern (5) beträgt ohne den allfälligen Überzug weniger als 20 μm und vorzugsweise höchstens 15 μm, so dass die Biegungen der Fasern (5), die beim in einem menschlichen oder tierischen Körper eingesetzten alloplastischen Material (1) auftreten, kaum mehr Ermüdungsbrüche bewirken.

Fig. 1

EP 0 312 494 A2

**Beschreibung**

## Alloplastisches Material für die Bildung eines künstlichen und/oder Verstärkung eines natürlichen Weichgewebeteils

Die Erfindung betrifft ein alloplastisches Material gemäss dem Oberbegriff des Anspruchs 1.

Es sind künstliche Bänder und Sehnen aus miteinander verwobenen oder verflochtenen Bündeln bekannt, die aus Kunststoff-Fasern oder mit einer resorbierbaren Substanz beschichteten Kohlenstoff-Fasern bestehen. Die Kunststoff-Fasern sind dabei 20 bis 30 µm und die Kohlenstoff-Fasern 7 bis 8 µm dick. Es sei hiezu zum Beispiel auf den Aufsatz "Implantatmaterialien für den alloplastischen Bandersatz" von L. Claes, C. Burri und R. Neugebauer in der 5. Vortragsreihe des Arbeitskreises Implantate, 13. November 1984, Seite 193, Deutscher Verband für die Materialprüfung e.V., verwiesen. Aus Kunststoff bestehende Fasern sind jedoch in einem Körper starken Alterungserscheinungen unterworfen, die vermutlich zumindest zum Teil durch die Aufnahme von Wasser aus der Körperflüssigkeit verursacht werden, so dass bei Kunststoff-Fasern im Verlauf der Zeit ein sogenanntes Kriechen, d.h. eine langsame Längenänderung, ein Festigkeitsabfall sowie eine Versprödung stattfinden kann. Die mechanischen Eigenschaften der zur Zeit überwiegend eingesetzten Fasern aus Kohlenstoff sind, bedingt durch die graphitische Struktur des Kohlenstoffs, ausgeprägt anisotrop. Biegungen mit kleinen Krümmungsradien oder auch nur relativ geringe Scherbeanspruchungen können daher einen Bruch der Faser verursachen, wie es auch aus der zitierten Publikation von Claes et al. hervorgeht. Ferner haben Kohlenstoff-Fasern nur eine geringe Duktilität und neigen aus diesen Gründen, insbesondere wenn ihre Beschichtung nach einem längeren Aufenthalt in einem menschlichen oder tierischen Körper aufgelöst wurde, zum Zerbröckeln.

Die Erfindung hat sich daher zur Aufgabe gestellt, ein allotropisches Material mit Fasern zu schaffen, das Nachteile der bekannten Materialien behebt und insbesondere auch Biegungen mit kleinen Krümmungsradien sowie verhältnismässig starke Scherbeanspruchungen ertragen kann und seine Festigkeit sowie Flexibilität auch dann noch beibehält, wenn es sich während einer verhältnismässig langen Zeitdauer in einem menschlichen oder tierischen Körper befindet.

Diese Aufgabe wird ausgehend von einem Material mit Fasern aus Kohlenstoff durch ein alloplastisches Material der einleitend genannten Art gelöst, das erfindungsgemäss durch den kennzeichnenden Teil des Anspruchs 1 gekennzeichnet ist.

Vorteilhafte Weiterbildungen des alloplastischen Materials gehen aus den vom Anspruch 1 abhängigen Ansprüchen hervor.

Die Erfindung betrifft ferner ein Verfahren für die Herstellung alloplastischen Materials, wobei das Verfahren gemäss der Erfindung durch die Merkmale des Anspruchs 7 gekennzeichnet ist. Vorteilhafte Ausgestaltungen des Verfahrens ergeben sich aus den von diesem Anspruch abhängigen Ansprüchen.

Das alloplastische Material kann nur ein einziges oder vorzugsweise zwei oder mehr Bündel von Fasern aufweisen. Das bzw. jedes Bündel kann mindestens 100, vorzugsweise mindestens 200 und beispielsweise mindestens oder ungefähr 1000 Fasern und bis beispielsweise höchstens 3000 Fasern enthalten.

Die zum gleichen Bündel gehörenden Fasern sollen beim sich im entspannten Zustand befindenden, noch nicht in einen menschlichen oder tierischen Körper eingesetzten, alloplastischen Material vorteilhafterweise nur locker und lose zusam mengehalten sein, so dass im allgemeinen Zwischenräume zwischen benachbarten Fasern vorhanden sind. Mit dem Begriff "im allgemeinen" ist hierbei gemeint, dass die Fasern mindestens bei Längsabschnitten, deren Summe einen wesentlichen Teil, nämlich mindestens etwa 60% und vorzugsweise mindestens etwa 80 % der gesamten Faserlänge beträgt, von der jeweils am nächsten benachbarten Faser durch einen freien Zwischenraum getrennt sind. Die Fasern können sich jedoch bei gewissen Stellen, beispielsweise bei Stellen, bei denen die Bündel wie bei den Längsrändern eines geflochtenen Bands stark gekrümmt sind, paar- oder gruppenweise berühren. Im übrigen können die Fasern eines Bündels parallel zu dessen Längsachse verlaufen oder um diese herum gedreht sein. Im letzteren Fall kann die Steigung der Fasern im Bündel mindestens gleich dem fünffachen und beispielsweise mindestens oder ungefähr gleich dem zehnfachen Durchmesser des Bündels sein.

Bei bevorzugten Ausführungsformen des alloplastischen Materials haben die Fasern ferner in diesem einen zickzack-und/oder wellen- und/oder wendelförmigen Verlauf. Falls das alloplastische Material nur ein einziges Bündel von Fasern aufweist, kann dieses Bündel zum Beispiel zickzack-, wellen-oder wendelförmig geformt und angeordnet werden. Wenn, wie es im allgemeinen der Fall ist, zwei oder mehr, zum Beispiel 8 bis 30 Bündel von Fasern vorhanden sind, können diese durch Flechten, Wirken, Weben oder Zusammendrehen locker und lose miteinander verbunden sein. Dabei können auch mehrere Verbindungsarten miteinander kombiniert werden. Die Bündel können durch diese Verbindungsarten die genannten zickzack-, wellen- oder wendelartigen Formen erhalten, wobei sie sich über ihre ganze Länge gegenseitig in regelmässigen Abständen oder unterbruchslos halten. Stattdessen können auch zwei oder mehr Bündel zickzack-, wellen- oder wendelförmig nebeneinander entlang von zueinander parallelen Mittellinien verlaufen, bei ihren Enden an gemeinsamen Befestigungsorganen befestigt, im übrigen aber unverbunden sein.

Das alloplastische Material kann beispielsweise eine längliche Form haben und zur Herstellung einer künstlichen Sehne oder eines künstlichen Bandes dienen. Falls das alloplastische Material eine definierte Längsrichtung hat, ist es günstig, wenn die Mittelachse der von jedem Bündel gebildeten Zickzacklinie, Welle oder Wendel parallel zur genannten Längsrichtung verläuft, so dass zumindest die den längenmässig grössten Teil jedes Bündels von Fasern bildenden Bündelabschnitte mit der genannten Längsrichtung Winkel bilden. Es können jedoch auch flächenhafte Fasergebilde hergestellt werden, die nicht notwendigerweise eine definierte Längsrichtung, sondern zum Beispiel einen polygonalen oder rundlichen Umriss haben, um beispielsweise Stücke eines Traggewebes, wie des Bauchfells, oder Stücke einer Membran oder anderen körperinneren Haut oder der Aussenhaut zu bilden.

Die Fasern oder, falls sie mit einer nichtmetallischen Hülle versehen sind, ihre Seelen oder Kerne, weisen ein metallisches Material mit mindestens einem Metall auf und können bei einer bevorzugten Ausführungsform aus einer Legierung bestehen, die zusätzlich zu dem das Basismetall bildenden Titan mindestens eines der Metalle, Niob, Tantal, Zirkon, Chrom, Molybdän, Eisen und Aluminium enthält. Der Titan-Anteil an der Legierung soll gewichtsmässig am grössten sein und im allgemeinen mindestens 50 Gew.-% betragen. Ferner sollen die Fasern abgesehen von allenfalls vorhandenen Verunreinigungen vorteilhafterweise keine anderen Metalle als Titan und die vorgängig genannten Legierungsbestandteile enthalten.

Das Titan ist reaktiv in Bezug auf Sauerstoff. Wenn sich nämlich zum Beispiel eine aus einer Titanlegierung bestehende Faser in einer freien oder gebundenen Sauerstoff enthaltenden, oxydierend wirkenden Umgebung befindet und etwa der Einwirkung von Luftsauerstoff oder von im Innern eines menschlichen oder tierischen Körpers in irgend einer Form, zum Beispiel als Be standteil elektrolytischer Körperflüssigkeiten vorhandenem Sauerstoff ausgesetzt ist, bildet sich an der Faseroberfläche eine filmartige, kompakte Metalloxydschicht, nämlich beim Titan $TiO_2$. Eine solche Oxydschicht gibt einen guten Korrosionsschutz für das sich unter ihr befindende Metall. Die Oxydschicht gibt insbesondere auch einen guten Schutz gegen die chemischen Einwirkungen, die von den Flüssigkeiten und sonstigen natürlichen Materialien eines menschlichen oder tierischen Körpers auf ein in einen solchen eingesetztes, alloplastisches Material ausgeübt werden können. Gemäss Untersuchungen an in menschlichen und tierischen Körpern eingesetzten Titanteilen erfolgt die Oxydation sehr langsam und schon bei einer oberflächennahen Schicht, nur mit einer Oxidationsrate von etwa $2 \cdot 10^{-5}$ µm/Tag. Der Abbau von 1 µm Material würde also mit der vorgenannten Oxydationsrate mindestens 50 000 Tage, d.h. mindestens 137 Jahre benötigen. Die tatsächlich zur Oxydation erforderliche Dauer ist zudem noch wesentlich höher, weil die oberflächliche Oxydschicht das darunter liegende Metall gegen korrosive Wirkungen und insbesondere auch gegen Oxydation schützt und dadurch das Weiterschreiten der Oxydation verlangsamt. Die Oxydation ist daher bei einem typischerweise 10 bis 15 µm betragenden Durchmesser der Fasern praktisch belanglos. Ähnliches gilt für die als mögliche Legierungskomponenten erwähnten, ebenfalls in Bezug auf Sauerstoff reaktiven Metalle Niob, Tantal, Zirkon, Chrom und Aluminium. Das ebenfalls noch als Legierungsbestandteil in Frage kommende Eisen ist zumindest als Bestandteil einer Titanbasislegierung korrosionsbeständig. Im weiteren haben das Titan und die genannten Legierungsmetalle beim Einsetzen in einen Körper auch keine toxischen Wirkungen. Das Titan und die genannten Legierungen auf Titanbasis sind also biologisch träge und nicht toxisch und lösen dementsprechend keine Gewebereaktionen aus. Ferner isoliert die oberflächliche Metalloxydschicht das darunterliegende Metall elektrisch gegenüber dem Körpergewebe.

Titan und die genannten Titanlegierungen haben auch eine gute Festigkeit und ein verhältnismässig niedriges Elastizitätsmodul, wobei die letztgenannte Eigenschaft besonders vorteilhaft ist, weil sie beim Biegen die Beanspruchung klein hält. Titan gehört zu den Metallen des sogenannten alpha-Typs. Titanlegierungen können abhängig von ihrer Zusammensetzung verschiedene Phasenstrukturen haben und je nach dem zum alpha-, alpha-beta- oder beta-Typ gehören. Die TiNbTaAl Legierung mit 3 Gew.-% Niob, 1 Gew.-% Tantal, 6 Gew.-% Aluminium und dem Rest Titan gehört zum Beispiel zum alpha-Typ. Zu den Legierungen des alpha-beta-Typs gehören zum Beispiel die TiAlFe-Legierung mit 5 Gew.-% Al, 2,5 Gew.-% Eisen und dem Rest Titan sowie die TiNbAl-Legierung mit 7 Gew.-% Niob, 6 Gew.-% Aluminium und dem Rest Titan. Zu den Legierungen des beta-Typs gehören die TiNb-Legierung mit 40 Gew.-% Niob und dem Rest Titan sowie die TiMoZrAl-Legierung mit 15 Gew.-% Molybdän, 5 Gew.-% Zirkon, 3 Gew.-% Aluminium und dem Rest Titan. Der Elastizitätsmodul liegt typischerweise für Titan sowie die Legierungen des alpha-Typs und des alpha-beta-Typs bei 100 bis 120 GPa und für die Legierungen des beta-Typs bei ungefähr 65 bis 110 GPa, wobei die Werte von der Wärmebehandlung abhängig sind. Die Legierungen des beta-Typs haben eine kubische Struktur und, wie auch in gewissem Ausmass diejenigen des alpha-beta-Typs, eine grössere plastische Verformbarkeit als Titan und die Legierungen des eine hexagonale Struktur aufweisenden alpha-Typs, so dass Legierungen des alpha-beta- und vor allem des beta-Typs vorteilhafter sind als Materialien des alpha-Typs. Zudem kann die Festigkeit bei den Legierungen des beta-Typs durch eine Wärmebehandlung, wie Glühen, insbesondere Lösungsglühen, und/oder Auslagern erhöht werden.

Die metallischen Fasern haben nach ihrer noch näher beschriebenen Herstellung eine verhältnismässig rauhe Oberfläche. Die rauhe Oberflächenstruktur ermöglicht ein gutes Festhaften des natürlichen Gewebes, welches das alloplastische Material nach dem Einsetzen in einen menschlichen oder tierischen Körper durchwächst. Das Einwachsen von Bindegewebe kann jedoch noch dadurch verbessert werden, dass man die metallische und/oder die von diesen gebildeten Bündel und/oder das aus der Gesamtheit mehrerer Bündel bestehende Gebilde mit einer organischen Substanz umhüllt, die im Körper von diesem und insbesondere dessen Flüssigkeiten resorbiert werden kann. Geeignete organische Substanzen, die in einem Körper innerhalb weniger Tage resorbiert werden, sind Kollagen, Polyglactin, Polylactat und Gelatine.

Die Fasern sollen auch dann, wenn ein sie enthaltendes, alloplastisches Material in einen menschlichen oder tierischen Körper über eine scharfe Kante verläuft oder aus anderen Gründen stark gekrümmt wird, nicht über die Elastizitätsgrenze hinaus beansprucht werden. Es wurde gefunden, dass sich diese Bedingung erfüllen

3

lässt, wenn der sogenannte kritische Krümmungsradius $r_c$ weniger als 1 mm und vorzugsweise höchstens oder ungefähr 0,5 mm beträgt. Hierbei wird unter dem kritischen Krümmungsradius $r_c$ der kleinste Krümmungsradius verstanden, mit dem die Fasern biegbar sind, ohne dass Brüche auftreten. Wenn die Fasern im Querschnitt kreisförmig sind und einen Durchmesser d haben und das Fasermaterial den Elastizitätsmodul E und bei Zugbeanspruchung die maximal zulässige Spannung $\sigma_z$ hat, kann man zeigen, dass der kritische Krümmungsradius gegeben ist durch die Formel:

$$r_c = \frac{E\,d}{2\,\sigma_z'} \qquad (1)$$

Für kaltverformtes Titan beträgt zum Beispiel die zulässige Spannung $\sigma_z$ = 0,9 GPa und der Elastizitätsmodul E = 105 GPa. Für eine kaltverformte TiNb-Legierung mit den weiter vorn angegebenen Gewichtsanteilen betragen $\sigma_z$ = 0,88 GPa und E = 69 GPa. Falls man einen kritischen Krümmungsradius von 0,5 mm vorgibt, ergibt sich für Titan ein Faserdurchmesser von ungefähr 9 μm und für die genannte TiNb-Legierung ein Faserdurchmesser d von ungefähr 13 μm. Wenn man anstelle der für Zugbeanspruchungen maximal zulässigen Spannung die niedrigere, für beliebig oft wiederholte Biegungen zulässige Ermüdungsspannung in die Formel (1) einsetzt, so sind die Faserdurchmesser noch um etwa 40% zu reduzieren.

Wenn die Fasern im Querschnitt kreisförmig sind, soll ihre Dicke, d.h. ihr Durchmesser, weniger als 20 μm und zum Beispiel höchstens oder ungefähr 15 μm sowie zum Beispiel mindestens 5 μm betragen. Zur Klarstellung sei noch bemerkt, dass unter der angegebenen Dicke der Fasern, im Fall, dass diese mit einer organischen Substanz überzogen sind, die Dicke im überzugsfreien Zustand, d.h. die Dicke der metallischen Seelen oder Kerne der Fasern zu verstehen ist. Im übrigen könnten die Fasern anstelle der bevorzugten, kreisrunden Querschnittsform möglicherweise eine andere, mehr oder weniger von einem Kreis abweichende, rundliche Querschnittsform haben. In einem solchen Fall soll unter der Dicke der Fasern deren maximale Querschnittsabmessung im überzugsfreien Zustand verstanden werden.

Wenn das alloplastische Material in einen menschlichen oder tierischen Körper eingesetzt ist, ermöglicht die lockere Struktur des Materials, dass Körperflüssigkeit zwischen die Fasern eindringen und natürliches Körpergewebe zwischen die Fasern hineinwachsen kann. Wenn die Fasern aus Titan oder einer titanhaltigen Legierung bestehen, bildet sich, wie bereits dargelegt, an den Aussenflächen der Fasern $TiO_2$. An diesen Ober-oder Aussenflächen können sich Hydroxidionen, Radikale mit einer Hydroxylgruppe und Radikale mit einer Aminogruppe sehr gut anlagern. Titan und seine genannten Legierungen zeigen daher ein bioaktives Verhalten, womit gemeint ist, dass sie nach dem Einsetzen des alloplastischen Materials in einen menschlichen oder tierischen Körper eine enge Bindung der natürlichen, weichen Gewebe und eventuell von fester Knochensubstanz des Körpers mit den Fasern ermöglichen und fördern, so dass das natürliche Gewebe an den Fasern festhaftet und gewissermassen anwächst. Es entsteht also quasi ein Verbundmaterial aus alloplastischem Material und natürlichem Gewebe, wobei das letztere eine Matrix bildet. Wenn trotz der guten Biegbarkeit der Fasern eine einzelne Faser des alloplastischen Materials brechen sollte, kann das an der betreffenden Faser festhaftende, natürliche, weiche Gewebe die Kraft auf die benachbarten Fasern übertragen und natürlich die Bruchstelle in Bezug auf die Kraftübertragung auch selbst überbrücken. Die Haftkraft, mit der das natürliche Gewebe an der Oberfläche der Fasern festhaftet, ist gleich dem Produkt Faseroberfläche mal Haftspannung $\sigma_h$. Wenn man unter der kritischen Abscher- oder Ausreisslänge L diejenige Länge eines Faserabschnitts versteht, bei der die Haftkraft gleich der Bruchfestigkeit einer Faser wird, ergibt sich

$$L = \frac{d\,\sigma_z}{4\,\sigma_h'} \qquad (2)$$

Experimentelle Untersuchungen über die Haftung von Knochen an rauhen Titanoberflächen ergaben Haftspannungen in der Grösse von 3 MPa. Wenn man annimmt, dass die Haftspannung für weiche Gewebe ungefähr die gleiche Grösse hat, ergibt sich zum Beispiel für Titanfasern mit 13 μm Durchmesser eine kritische Ausreisslänge L in der Grösse von ungefähr oder etwas weniger als 1 mm. Wenn der Durchmesser d der Fasern, wie bereits erwähnt, weniger als 20 μm und beispielsweise 5 bis 15 μm ist, kann also einerseits ein ausreichend kleiner, kritischer Krümmungsradius und andererseits eine Ausreisslänge L erzielt werden, die deutlich grösser ist als der Abstand benachbarter Fasern in einem Bündel lose zusammengehaltener Fasern.

4

Dieses letzte Merkmal trägt beim allfälligen Bruch einer einzelnen Faser zu einer guten Kraftübertragung von der gebrochenen Faser auf die benachbarten Fasern bei.

Wenn man natürliche, menschliche oder tierische Gewebe, wie Bänder, Faszien und Sehnen bis zum Auftreten eines Bruchs streckt und die Zugkraft als Kurve aufzeichnet, nimmt die Steigung der Kurve anfänglich zu und bleibt danach konstant, bis die Kurve abflacht und nach dem Erreichen der Reissfestigkeit, d.h. der maximalen Zugkraft bis zum Stattfinden des Bruchs wieder ein wenig abfällt. Für eine einzelne metallische Faser steigt hingegen die entsprechende Kurve von Anfang an linear an, bis der Bereich plastischer Deformation erreicht wird und die Kurvensteigung ein wenig abnimmt, aber bis zum Bruch immer noch positiv bleibt. Abgesehen von diesen verschiedenen Kurvenformen unterscheiden sich die Festigkeits- und Dehnungseigenschaften der metallischen Fasern auch in quantitativer Hinsicht stark von denjenigen natürlicher Gewebe. Während der Elastizitätsmodul für natürliche Gewebe wie Sehnen und Bänder, im linearen Kurventeil im Grössenbereich von 0,5 bis 1,8 GPa liegt, beträgt er für Fasern aus Titan, wie bereits erwähnt, 105 GPa und für Titanlegierungen der genannten Typen ungefähr 65 bis 120 GPa. Die maximal zulässige Zugspannung beträgt für die genannten, natürlichen Gewebe etwa 60 bis 110 MPa, für reines Titan 800 MPa und für Titanlegierungen 800 bis 1400 MPa. Vor allem aber sind natürliche, weiche Gewebe der genannten Art typischerweise im elastischen Deformationsbereich etwa 10% und bis zum Bruch ungefähr 15% dehnbar, während metallische Fasern im elastischen Deformationsbereich nur etwa 1% dehnbar sind.

Die Fasern können jedoch in der weiter vorn erwähnten Weise locker miteinander verbunden und derart angeordnet werden, dass sie zickzack-, wellen- und/oder wendelförmig verlaufen. Bei einem eine definierte Längsrichtung aufweisenden, etwa zur Bildung einer Sehne oder dergleichen dienenden, alloplastischen Material können die Fasern dann mindestens im allgemeinen schief zu dieser Längsrichtung und damit zu derjenigen Richtung verlaufen, entlang der das alloplastische Material bei seiner Verwendung eine Zugkraft übertragen soll. Bei einem flächenhaften, zur Bildung eines Bauchfells oder dergleichen dienenden, alloplastischen Material können dessen Fasern mindestens stellenweise einen Winkel mit jeder Richtung bilden, entlang der bei der Verwendung des alloplastischen Materials eine Kraft zu übertragen ist. Die einzelnen Fasern können sich dann beim Deformieren des alloplastischen Materials bezüglich einander begrenzt bewegen. Das alloplastische Material erhält dadurch eine gewisse Formelastizität. Diese wird nach dem Einsetzen in einen menschlichen oder tierischen Körper noch wesentlich dadurch vergrössert, dass natürliches, weiches Gewebe, wie Bindegewebe, durch die Zwischenräume zwischen den Fasern hindurchwächst. Durch geeignete Ausbildung kann erreicht werden, dass das alloplastische Material, ausgehend von seinem entspannten Zustand, unter Änderung des Verlaufs der Fasern zumindest nach seiner Durchwachsung durch natürliches Gewebe mindestens in einer Richtung um mindestens und vorzugsweise mehr als 5%, beispielsweise um mindestens 10% und im Bedarfsfall mindestens 15% oder sogar mindestens 20% dehnbar ist, ohne dass die Fasern nennenswert plastisch derformiert werden und natürlich auch ohne dass die Fasern brechen.

Wenn das in einem menschlichen oder tierischen Körper eingesetzte, alloplastische Material von natürlichem Gewebe durchwachsen ist, entsteht ein Verbundmaterial, wobei die auf dieses ausgeübten Kräfte teils von den metallischen Fasern, teils vom natürlichen Gewebe aufgenommen werden. Der wirksame Elastizitätsmodul ist in diesem Fall zumindest in der Anfangsphase eines Dehnungsvorganges näherungsweise gleich dem gewichteten Mittel zwischen dem Elastizitätsmodul des alloplastischen Materials und dem Elastizitätsmodul des natürlichen Weichgewebes, wobei die Gewichtungsfaktoren für die Mittelwertbildung proportional zu den Anteilen der Summe der Faserquerschnittsflächen bzw. der Summme der Weichgewebequerschnittsflächen an der gesamten Querschnittsfläche des Verbundmaterials sind. Im übrigen ermöglicht die lockere Struktur des alloplastischen Materials, dass an ihm angreifende Zugkräfte verhältnismässig gleichmässig auf viele Fasern verteilt werden. Erst wenn die formelastische Dehnbarkeit des Verbundmaterials ausgeschöpft ist, beginnen sich die Fasern selbst nennenswert zu dehnen, wobei der Elastizitätsmodul dann grösser wird. Obschon sich also die metallischen Fasern und das natürliche Gewebe in Bezug auf Festigkeits- und Deformationseigenschaften stark unterscheiden, kann mit metallischen Fasern ein alloplastisches Material hergestellt werden, das als Ganzes, insbesondere nach dem Einwachsen von natürlichem, weichen Gewebe, einen qualitativ ähnlichen Zusammenhang zwischen Zugspannung und Dehnung ergibt, wie das ursprünglich vorhandene, natürliche Gewebe.

Bei einem band- oder schlauchförmigen Geflecht kann der Flechtwinkel, d.h. der Winkel zwischen einem Faserbündel und der Längsrichtung des Geflechts, zur Erzielung einer mindestens etwa 10% betragenden, formelastischen Dehnbarkeit abhängig von der Dichte und dem Füllgrad des Geflechts 30° bis 60° und zum Beispiel 40° bis 50° betragen. Wenn das alloplastische Material zum Beispiel nur ein einzelnes zickzack- oder wellenförmig entlang einer Ebene verlaufendes Faserbündel oder mehrere mit der genannten Form mindestens einigermassen frei nebeneinander verlaufende Faserbündel aufweist und eine formelastische Dehnbarkeit von mindestens oder mehr als 5% und beispielsweise ungefähr oder mindestens 10% gewünscht wird, kann das Verhältnis zwischen der Wellenlänge und der Wellenhöhe beispielsweise ungefähr 4 oder weniger betragen. Falls ein oder jedes Faserbündel für sich alleine eine Wendel bildet, kann das Verhältnis zwischen Steigung und Durchmesser zur Erzielung einer Dehnbarkeit von mindestens oder mehr als 5% und beispielsweise ungefähr oder mindestens 10% zum Beispiel in der Grösse von 6 oder weniger liegen.

Man kann durch rechnerische Analysen ungefähr abschätzen, wie sich die Kräfte in einem Verbundmaterial, das aus metallischen Fasern und die Zwischenräume zwischen diesen ausfüllenden, natürlichem Gewebe besteht, auf die Fasern und das natürliche Gewebe verteilen und welche Kräfte die Fasern und das Gewebe bis zum Bruch aufnehmen können. Diese Analysen zeigen, dass das natürliche Gewebe, wenn sein Anteil am Gesamtvolumen des Verbundmaterials etwa 90 bis 99% erreicht, mindestens den gleichen Anteil an der Gesamtkraft aufnimmt wie die Fasern und bis zum Bruch auch mindestens eine ähnliche Maximalkraft aufnehmen kann wie die Fasern. Dies bedeutet, dass zum Beispiel nach dem Einsetzen eines als künstliches Band dienenden, alloplastischen Materials soviel natürliches Gewebe in dieses hineinwachsen kann, dass quasi ein neues Band aus natürlichem Gewebe entsteht, in welchem das alloplastische Material praktisch nur noch als Leitgerüst für das nachwachsende Gewebe dient.

Die totale Querschnittsfläche eines Bündels von Fasern und das Verhältnis V zwischen der Summe der Querschnittsflächen der zum gleichen Bündel gehörenden Fasern und der totalen Querschnittsfläche des Bündels können entlang dem Bündel variieren. Wenn ein längliches, alloplastisches Material mit einem oder mehreren Faserbündeln für die Verwendung mit seinen Enden an Körperteilen befestigt wird, werden die Fasern an den Enden zudem bei den Befestigungsstellen natürlich zusammengedrückt. Ferner können die Fasern auch zusammengezogen werden, wenn das alloplastische Material bei seiner Benutzung als künstliches Band oder als künstliche Sehne einer Zugbeanspruchung aus gesetzt wird. Wenn das alloplastische Material nach seinem Einsetzen in einen menschlichen Körper von natürlichem Gewebe durchwachsen wird, kann es zudem durch dieses einwachsende Gewebe deformiert werden, wodurch zum Beispiel die Gesamt-Querschnittsfläche vergrössert werden kann. Obschon also die Abstände benachbarter Fasern nach dem Einsetzen des alloplastischen Materials nicht mehr identisch mit denjenigen vor dem Einsetzen sind, wird das sich nach dem Anwachsen von natürlichem Gewebe ergebende Verhältnis zwischen dem gesamten Volumen der Fasern und dem gesamten Volumen des eingewachsenen Gewebes doch stark durch die Abstände der Fasern im entspannten alloplastischen Material vor dem Einsetzen beeinflusst. Damit das alloplastische Material ausreichend von natürlichem Gewebe durchwachsen werden kann, dürfte es daher günstig sein, wenn das Verhältnis V beim noch nicht in einen Körper eingesetzten alloplastischen, sich im entspannten Zustand befindenden Material höchstens 0,5, vorzugsweise höchstens 0,2, beispielsweise höchstens oder ungefähr 0,1 und beispielsweise mindestens oder ungefähr 0,01 beträgt. Diese Grenzwerte sollen vorzugsweise auch für den Mittelwert des Verhältnisses V gelten, wobei unter dem Mittelwert der arithmetisch über die gesamte Länge des Faserbündels des entspannten und unbefestigten alloplastischen Materials gemittelte Mittelwert gemeint ist.

Metallische Fasern mit Durchmessern von weniger als 20 μm sind kaum noch mit zur Herstellung einzelner Drähte gebräuchlichen Verfahren herstellbar. Solche Fasern lassen sich jedoch bündelweise herstellen, indem vorzugsweise einen kreisförmigen Querschnitt aufweisende, verhältnismässig dicke, aus dem Material der zu bildenden Fasern bestehende Drähte, deren Anzahl mit der gewünschten Anzahl Fasern eines Bündels übereinstimmt, in Bohrungen eines Blocks eingesetzt werden, der aus einem anderen metallischen Material besteht als die Fasern. Das Material des eine Matrix bildenden Blocks soll vorzugsweise weicher sein als das Fasermaterial und in Bezug auf Verlängerungs-Verformungen mindestens eine ähnliche Duktilität wie das letztere haben. Die Matrix kann zum Beispiel aus Kupfer oder einer Kupfer-Nickel-Legierung bestehen. Der aus einer Matrix und den darin eingebetteten Drähten bestehende Verbund-Rohling kann durch warmes oder kaltes Verformen, wie Pressen und/oder Walzen und/oder Ziehen stufenweise gestreckt und im Durchmesser reduziert werden, so dass die Dicke der Drähte auf die gewünschte Dicke der Fasern abnimmt. Vorzugsweise ist zumindest der Schlussteil bzw. die Schlussphase des Verformungsvorgangs eine Kaltverformung. Die Verformung kann zum Beispiel durch Warmpressen und anschliessendes Kaltziehen erfolgen. Bei einem solchen eine Querschnittsflächenverminderung sowie Verlängerung des Rohlings und der Drähte bewirkenden Verformungsvorgang bleibt das Volumen des Rohlings sowie der einzelnen Drähte erhalten. Ein ähnliches Verformungsverfahren ist an sich für die Herstellung von Supraleitern mit in eine Kupfermatrix eingebetteten Fasern aus einer Titan-Niob-Legierung bekannt. Während bei den fertigen Supraleitern die Matrix erhalten bleibt, wird diese für die Bildung von Fasern für ein alloplastisches Material nach der Verformung mit einer das Fasermaterial nicht angreifenden Säure, zum Beispiel Salpetersäure herausgelöst.

Wie bereits erläutert, soll das bzw. jedes Faserbündel des fertiggestellten, alloplastischen Materials vorzugsweise einen zickzack-, wellen- und/oder wendelförmigen Verlauf haben, wobei zum Beispiel mehrere Bündel ein Geflecht, Gewirke, Gewebe und/oder einen Schlauch und/oder ein Seil bzw. einen Zwirn bilden. Ein solcher Verlauf des bzw. jedes Faserbündels kann durch einen Formgebungsvorgang erzielt werden, bei dem die Fasern zumindest im wesentlichen nur noch gebogen werden, d.h. im Gegensatz zur vorher bei ihrer Herstellung aus Drähten stattgefundenen Verformung nicht mehr oder zumindest nicht mehr wesentlich dünner und länger gemacht werden. Das bzw. jedes Faserbündel kann bei der genannten, ohne oder zumindest ohne wesentliche Querschnittsflächenverminderung sowie ohne Verlängerung der Fasern stattfindenden Formgebung bereits vor dem Herauslösen der Matrix zusammen mit dieser oder nach dem Herauslösevorgang verdrillt und/oder entsprechend der gewünschten Art des herzustellenden, alloplastischen Materials als einzelnes Bündel zickzack-, wellen- oder wendelförmig geformt und/oder mit anderen Faserbündeln verbunden werden. Wenn die Verdrillung und/oder Verflechtung und/oder sonstige Formgebung der Faserbündel vor dem Herauslösen der Matrix erfolgt, gewährleistet die feste Verbindung zwischen den Fasern und der Matrix, dass die Fasern ohne zu brechen gleich wie die sie umgebende Matrix gebogen werden. Die Duktilität und/oder Festigkeit der Fasern kann nach deren durch eine volumenerhaltende, verlängernde Querschnittsflächenverminderung von Drähten erfolgten Herstellung vor und/oder während und/oder nach dem Herauslösen der Matrix sowie vor und/oder während und/oder nach ihrer in der beschriebenen Weise ohne wesentliche Verlängerung und Querschnittsflächenveränderung stattfindenden Formgebung der Fasern auch noch durch eine Wärmebehandlung, wie Glühen, etwa Weichglühen oder Lösungsglühen, und/oder Auslagern, in erwünschter Weise beeinflusst werden. Falls die Fasern zum Beispiel aus einer Titanlegierung des beta-Typs bestehen, und insbesondere falls zudem bei der zur Herstellung durch Verlängern und Querschnittsverminderung dienenden, mehrstufigen Verformung zumindest am Ende eine Kaltverformung stattfindet, kann die Festigkeit der Fasern noch durch eine Wärmebehandlung, wie Lösungsglühen und/oder Auslagern, erhöht werden. Ferner kann man die Fasern sofort nach der Herstellung eines Bündels von Fasern oder erst nach dessen zickzack-, wellen- oder wendelförmiger Formgebung und/oder Verbindung mit anderen Faserbündeln, aber selbstverständlich erst nach dem Herauslösen der Matrix mit einer der erwähnten, organischen Substanzen beschichten.

Der Erfindungsgegenstand soll nun anhand in der Zeichnung dargestellter Ausführungsbeispiele erläutert werden. In der Zeichnung zeigen

die Figur 1 eine Draufsicht auf ein alloplastisches Material in der Form eines ebenen Geflechts,

die Figur 2 einen schematisierten Querschnitt durch einen Teil eines Bündels von Fasern in grösserem Masstab,

die Figur 3 eine schematisierte Endansicht eines schlauchförmigen, alloplastischen Materials,

die Figur 4 eine schematisierte Ansicht eines alloplastischen Materials mit einem wendelförmigen Bündel von Fasern und

die Figur 5 eine schematisierte Ansicht eines alloplastischen Materials mit einem zickzack- oder wellenförmigen Bündel von Fasern.

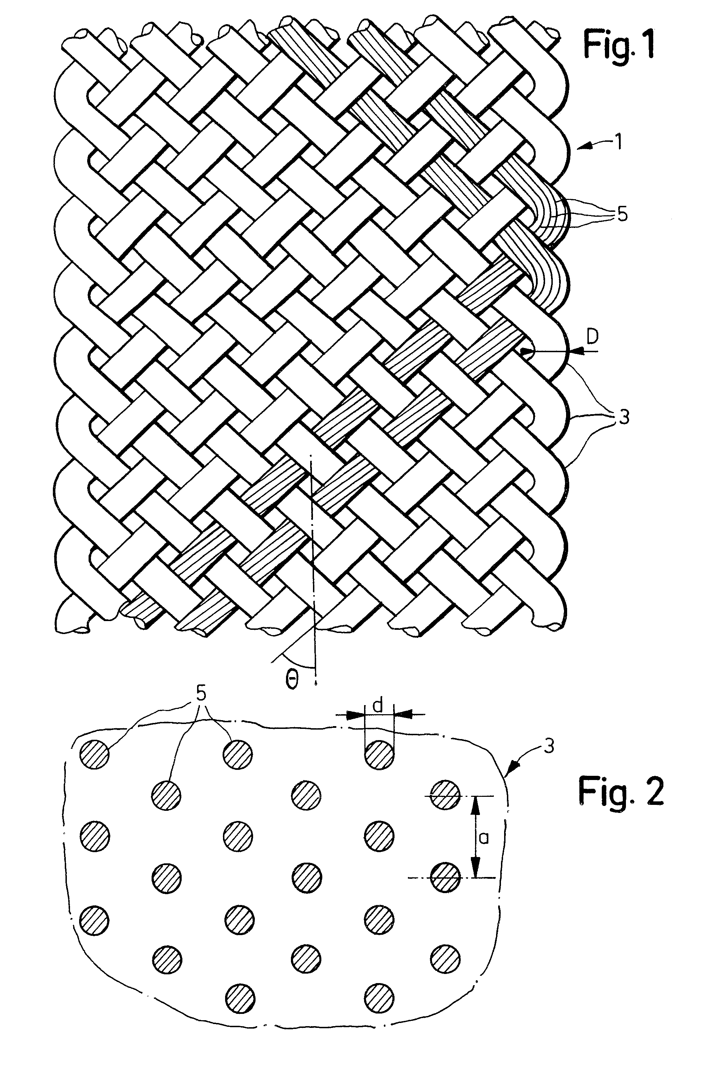

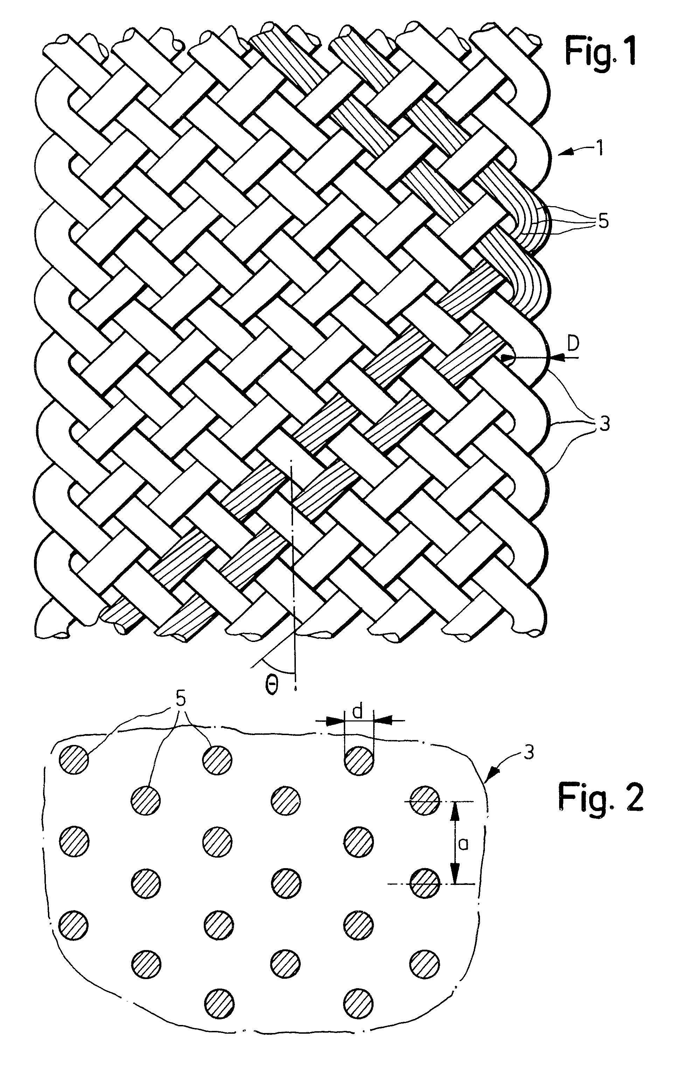

Das in der Figur 1 dargestellte, längliche, alloplastische Material 1 ist durch ein flächenhaftes, einschichtiges Band gebildet und weist eine Anzahl Bündel 3 auf, die lose miteinander verbunden, nämlich lose miteinander verflochten sind. Jedes Bündel 3 verläuft also zickzack- oder wellenförmig entlang einer Ebene, wobei aber die Bündel durch die Verflechtung über ihre ganze Länge in regelmässigen Abständen miteinander verbunden sind. Jedes Bündel 3 besteht aus lose zusammengehaltenen Fasern 5. Diese wurden in der Figur 1 nur bei zwei Bündeln angedeutet, wobei die Anzahl Fasern in Wirklichkeit wesentlich grösser ist, als es gezeichnet wurde. Jede Faser besteht aus mindestens einem metallischen Material, nämlich Titan oder einer Legierung auf Titanbasis.

Die Bündel 3 bilden mit der Längsrichtung des Bandes den zum Beispiel 45° betragenden Flechtwinkel θ und können beispielsweise eine im wesentlichen kreisrunde Querschnittsform haben, wobei der Durchmesser eines Bündels 3 mit D bezeichnet ist. Die Fasern 5 haben, wie in der Figur 2 ersichtlich, eine kreisrunde Querschnittsform mit dem Durchmesser d. Die zum gleichen Bündel 3 gehörenden Fasern 5 sind lose miteinander verdrillt, so dass im allgemeinen Zwischenräume zwischen benachbarten Fasern vorhanden sind. Die Fasern können sich jedoch bei gewissen Stellen, beispielsweise bei Stellen, bei denen die Bündel 3 wie bei den Längsrändern des Bandes stark gekrümmt sind, paar- oder gruppenweise berühren. Die Fasern 5 können sich dann mindestens beim sich in entspanntem Zustand befindenden Band im allgemeinen quer zur Faser- und Bündellängsrichtung bezüglich einander bewegen. Da die Bündel 3 einen gekrümmten Verlauf haben, ermöglicht die lockere Struktur des Fasergebildes, dass Längsabschnitte der Fasern 5 sich auch in der Faserlängsrichtung in gewissen Grenzen bezüglich einander bewegen können.

Die Abstände der einander am nächsten benachbarten Fasern können von Ort zu Ort und von Faser zu Faser variieren. Um einige quantitative Grössen und Bezeichnungen erläutern zu können, wird nun jedoch angenommen, der Abstand der einander am nächsten benachbarten Fasern habe für alle Fasern den Wert a, wobei man diesen Wert a als einen in einer zweckmässigen Weise gemittelten Mittelwert auffassen kann. Damit sich zwischen benachbarten Fasern freie Zwischenräume ergeben, muss der Abstand a natürlich grösser sein als der Durchmesser d der Fasern. Beim Zeichnen der Figur 2 wurde angenommen, dass die Fasern 5 in einem quer zum betreffenden Bündel 3 verlaufenden Querschnitt auf ein zweidimensionales, regelmässiges, hexagonales Gitter verteilt und in den Ecken und Zentren zusammenhängender, gleich grosser Sechsecke angeordnet sind. Der mit a bezeichnete mittlere Abstand der einander paarweise am nächsten benachbarten Fasern ist dann gleich der Seitenlänge eines Sechsecks. Wenn man annimmt, dass die Fasern gemäss der Darstellung in der Figur 2 ein hexagonales Gitter definieren, habe das Verhältnis V den Wert $V_{Sechseck}$, so dass gilt,

$$V = V_{Sechseck} = 0,906 \ d^2/a^2 \qquad (3)$$

Die Fasern könnten auch stellenweise ein zweidimensionales, quadratisches Gitter aufspannen, d.h. in einem quer zur Bündellängsrichtung gelegten Querschnitt auf den Ecken zusammenhängender, gleich grosser Quadrate angeordnet sein. In diesem Fall würde dann $V = V_{Quadrat}$, so dass gilt,

$$V = V_{Quadrat} = 0,785 \ d^2/a^2 \qquad (4)$$

Wenn man also beispielsweise den Durchmesser d und das Verhältnis V vorgibt, kann man mit den Formeln (3) und (4) die ungefähre Grösse des mittleren Abstandes a berechnen oder mindestens abschätzen. Im Fall der Figur 2 beträgt V ungefähr 0,1 und d/a dementsprechend ungefähr 0,3.

Für die Herstellung des alloplastischen Materials 1 kann man zunächst gemäss dem in der Einleitung beschriebenen Verfahren Bündel von Fasern herstellen, die letzteren miteinander verdrillen, d.h. um die Längsachse des Bündels herumdrehen und danach die gewünschte Anzahl Bündel miteinander verflechten. Stattdessen kann man bereits die gestreckten, zusätzlich zu Fasern noch eine zum Beispiel aus Kupfer bestehende Matrix enthaltenden Verbunddrähte verdrehen sowie miteinander verflechten und die Matrix erst nach der Verflechtung mit einer Säure herauszulösen. Ferner können die Faserbündel vor oder nach ihrer Verflechtung nach dem Herauslösen der Matrix mit einer organischen, im Körper später resorbierbaren Substanz überzogen werden. Diese umschliesst dann auch die einzelnen Fasern mit Ausnahme von allenfalls vorhandenen, ohne Zwischen räume aneinander anliegenden Faserabschnitten. Man kann jedoch auch auf ein Verdrehen bzw. Verdrillen der zum gleichen Bündel gehörenden Fasern verzichten, so dass alle Fasern eines Bündels mehr oder weniger parallel zu dessen Längsachse verlaufen. Nach dem Verflechten der Bündel werden die zum gleichen Bündel gehörenden Fasern dann infolge der Verflechtung zusammengehalten. Falls die Fasern aus einer Titanlegierung des beta-Typs hergestellt werden, können ihre Festigkeitseigenschaften vor oder nach der Verflechtung der Faserbündel noch durch Lösungsglühen und/oder Auslagern verbessert werden.

Das längliche, alloplastische Material 1 kann zur Bildung eines künstlichen Bandes oder einer künstlichen Sehne beim Einsetzen ein einem menschlichen oder tierischen Körper mit seinen Enden an Knochen befestigt werden. Diese Befestigung kann beispielsweise dadurch erfolgen, dass Löcher in die Knochen gebohrt und die Fasergebilde mit in die Löcher eingesetzten, beispielsweise ebenfalls aus Knochenmaterial bestehenden Zapfen festgeklemmt werden. Das natürliche, feste Knochenmaterial kann dann analog wie das natürliche Weichgewebe gewissermassen an den metallischen Fasern anwachsen.

Für Versuche wurden Rohlinge mit 1800 aus der zum beta-Typ gehörenden Ti-Nb-Legierung mit 40 Gew.-% Niob und dem Rest Titan bestehenden, in eine Kupfer-Matrix eingebetteten Drähten gebildet. Aus den Rohlingen wurden durch Warmpressen und Kaltziehen Verbunddrähte mit Durchmessern von 0,8 mm sowie Längen von 10 bis 15 m gebildet und danach auf die gewünschte Länge zugeschnitten. Die nach dem Herauslösen der Kupfermatrix vorhandenen Fasern hatten Durchmesser von 12 bis 13 $\mu$m. Die in einem Bündel vorhandenen Fasern hatten daher eine Gesamtquerschnittsfläche von etwa 0,22 mm$^2$. Die Zugfestigkeiten der Bündel lagen zwischen 150 und 200 N. Zur Bildung eines alloplastischen Materials wurden 18 Bündel zu einem Band verflochten, das dann eine Zugfestigkeit von etwa 3 kN hatte. Dies ist wesentlich mehr als zum Beispiel die Reissfestigkeit eines natürlichen Kniebandes.

Auf gleiche Weise wurden auch Faserbündel aus der zum beta-Typ gehörenden Legierung mit 15 Gew.-% Molybdän, 5 Gew.-% Zirkon, 3 Gew.-% Aluminium und dem Rest Titan gebildet. Die Legierung ist ähnlich wie die TiNb-Legierung ohne störende Verfestigung gut verformbar. Die Faserbündel wurden vor oder nach dem Herauslösen der Matrix einer Wärmebehandlung bei etwa 500°C unterzogen. Bei Zerreissversuchen wurden Spannungen bis 1,4 GPa ermittelt.

Wenn das ein künstliches Band oder eine künstliche Sehne bildende, alloplastische Material in einen menschlichen oder tierischen Körper eingesetzt wird, bildet sich nach einer gewissen Zeit eine die Fasern 5 durchdringende und umschliessende Matrix von natürlichem, an den Fasern 5 anhaftendem Weichgewebe. Dieses wird beim Dehnen des Fasergebildes 1 ebenfalls gedehnt, wobei sich die Kräfte auf das Fasergebilde 1 und das natürliche Weichgewebe verteilen. Wenn die Länge eines Faserabschnitts, an dem natürlichen Weichgewebe anhaftet, mindestens gleich der in der Einleitung definierten, kritischen Ausreisslänge L ist, wird die zum Abscheren des natürlichen Weichgewebes von den Fasern 5 erforderliche Scherkraft grösser als die Bruchfestigkeit der Faser bei Zugbeanspruchung. Gemäss der Formel (2) liegt die kritische Ausreisslänge für Fasern aus der genannten Titan-Niob-Legierung mit 12 bis 13 $\mu$m Durchmesser in der Grösse von etwa 0,6 bis 1 mm. Dies ist im Vergleich zur Länge üblicher Bänder und Sehnen kurz, so dass also das natürliche Weichgewebe, sobald es auch nur einen kurzen Teil des alloplastischen Materials durchwachsen hat, ungleiche Beanspruchungen der Fasern ausgleichen und beim allfälligen Bruch einer Faser die von dieser übertragenen Kraft auf die Nachbarfasern übertragen kann. Wenn das Verhältnis V, d.h. der Anteil der totalen Querschnittsfläche der Fasern 5 eines Bündels 3 an der gesamten Querschnittsfläche des von Gewebe durchwachsenen Bündels zum Beispiel etwa 0,1 beträgt, so dass sich etwa das in der Figur 2 dargestellte Verhältnis zwischen den Abständen a benachbarter Fasern und dem Durchmesser d ergibt und die Abstände a im Mittel etwa 0,04 mm betragen, sind einerseits die Abstände a noch wesentlich kleiner als die kritische Ausreisslänge L, was eine gute Kraftübertragung zwischen benachbarten Fasern ermöglicht, und andererseits der Volumenmanteil des nachgewachsenen, natürlichen Gewebes genügend gross, damit dieses quasi die volle Funktion eines Bandes oder einer Sehne ausüben kann.

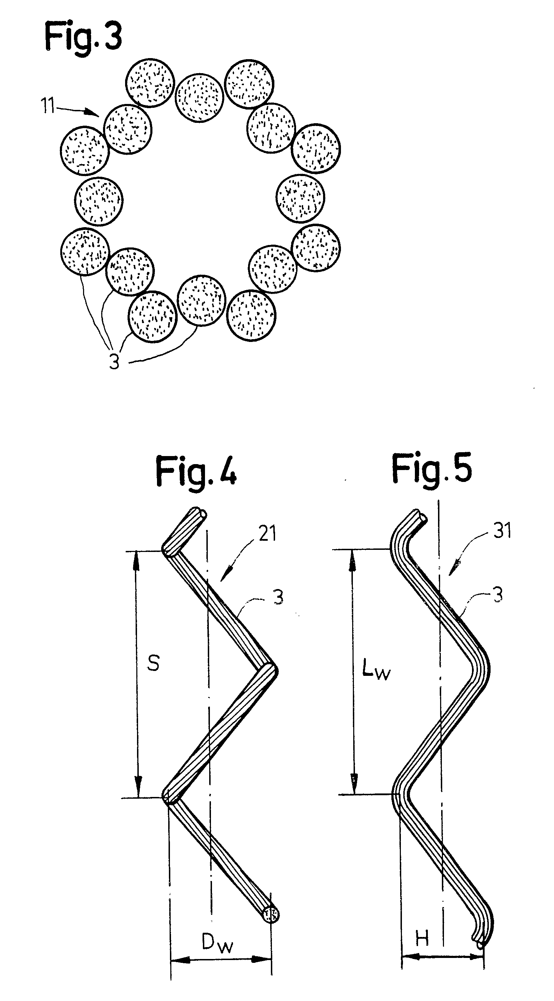

Die in der Figur 3 dargestellte Variante des alloplastischen Materials 11 besitzt eine Anzahl Bündel 3 mit Fasern, wobei die einzelnen Bündel zu einem Schlauch verflochten sind. Das Geflecht ist vorzugsweise derart beschaffen, dass alle Bündel 3 mit gleicher Steigung um die Längsachse des Schlauchs herum verlaufende Wendeln bilden, wobei die eine Hälfte der Bündel in der Drehrichtung einer rechtsgängigen und die andere Hälfte der Bündel in der Drehrichtung einer linksgängigen Schraubenlinie verlaufen.

Das in der Figur 4 dargestellte, alloplastische Material 21 weist ebenfalls mindestens ein wendelförmiges Bündel 3 von beispielsweise leicht verdrillten Fasern auf. Beim alloplastischen Material 21 ist jedoch das Bündel 3 mit keinem anderen Bündel verflochten, sondern höchstens bei seinen Enden durch nicht dargestellte Befestigungsorgane mit anderen Bündeln verbunden und also über den grössten Teil seiner Länge frei. Die Steigung S ist grösser als der Durchmesser der Wendel oder, genauer gesagt, deren mittlerer Durchmesser $D_w$ und beträgt zum Beispiel höchstens oder ungefähr 6 $D_w$.

Das in der Figur 5 dargestellte, alloplastische Material 31 weist mindestens ein Bündel 3 von Fasern auf, das zickzack- und/oder wellenförmig entlang einer Ebene verläuft. Die Wellenlänge $L_w$ ist grösser als die Wellenhöhe H des Bündels oder, genauer gesagt, der Bündel-Mittelachse und zum Beispiel ungefähr oder höchstens 4 H. Falls mehrere Bündel vorhanden sind, sollen diese höchstens bei ihren Enden über gemeinsame Befestigungsorgane miteinander verbunden sein.

Zur Herstellung wendel- oder wellenförmiger Bündel gemäss der Figur 4 bzw. 5 kann man ein Bündel in eine Matrix eingebetteter Drähte aus Titan oder einer Titanlegierung in der einleitend beschriebenen Art verformen, bis die Drähte die für die Fasern gewünschten Durchmesser haben. Der so gebildete Verbunddraht kann nun zu einer Wendel bzw. Welle geformt werden. Danach kann das die Matrix bildende Metall mit einer Säure herausgelöst werden.

**Patentansprüche**

1. Alloplastisches Material für die Bildung eines künstlichen und/oder Verstärkung eines natürlichen Weichgewebeteils, zum Beispiel eines Bandes, einer Sehne oder eines Traggewebes, eines menschlichen oder tierischen Körpers mit Fasern (5), deren Dicke weniger als 20 Mikrometer beträgt, dadurch gekennzeichnet, dass die Fasern (5) ein metallisches Material aufweisen.

2. Material nach Anspruch 1, dadurch gekennzeichnet, dass das metallische Material der Fasern (5) zum Teil aus Titan, vorzugsweise einer Legierung des alpha-beta- oder des beta-Typs, besteht, wobei das Titan vorzugsweise die metallische Komponente mit dem gewichtsmässig grössten, zum Beispiel mindestens 50 Gew.-% betragenden Anteil an der Legierung ist.

3. Material nach Anspruch 2, dadurch gekennzeichnet, dass die Fasern (5) zusätzlich zu Titan noch mindestens eines der Metalle Niob, Tantal, Zirkon, Chrom, Molybdän, Eisen und Aluminium enthalten.

4. Material nach einem der Ansprüche 1 bis 3, dadurch gekennzeichnet, dass die einzelnen Fasern (5) und/oder ein Bündel (3) von Fasern (3) und/oder ein mehrere Bündel (3) aufweisendes Gebilde mit einer in einem menschlichen und/oder tierischen Körper durch diesen resorbierbaren Substanz, zum Beispiel Kollagen, Polyglactin, Polylactat oder Gelatine umhüllt sind bzw. ist.

5. Material nach einem der Ansprüche 1 bis 4, dadurch gekennzeichnet, dass es mindestens ein Bündel (3) mit mindestens 100, vorzugsweise mindestens 200 und beispielsweise mindestens oder ungefähr 1000 Fasern (5) aufweist, dass die zum gleichen Bündel (3) gehörenden Fasern (5) nur lose zusammengehalten sind, so dass nach dem Einsetzen des alloplastischen Materials in einen menschlichen oder tierischen Körper mindestens zwischen Abschnitten der einander benachbarten Fasern (5) natürliches Gewebe hindurchwachsen kann, wobei das bzw. jedes Bündel (3) vorzugsweise zickzack- und/oder wellen-und/oder wendelförmig ist, wobei vorzugsweise mindestens zwei Bündel (5) vorhanden sind und zum Beispiel lose und/oder locker durch Flechten, Wirken, Weben oder Zusammendrehen miteinander verbunden sind, so dass sie einen Schlauch oder ein Seil oder ein zusammenhängendes, flächenhaftes Gebilde, insbesondere ein Band, bilden, oder zum Beispiel nebeneinander zickzack-, wellen- oder wendelförmig angeordnet und bei ihren Enden an gemeinsamen Befestigungsorganen gehalten, im übrigen aber unverbunden sind, wobei die zum gleichen Bündel (3) gehörenden Fasern (5) zum Beispiel parallel zur Längsachse des Bündels (3) verlaufen oder um diese herum gedreht sind.

6. Material nach Anspruch 1, dadurch gekennzeichnet, dass die Fasern (5) derart verlaufen, dass das Material ausgehend von seinem entspannten Zustand unter einer Änderung des Verlaufs der Fasern (5) mindestens in einer Richtung um mindestens oder vorzugsweise mehr als 5% und beispielsweise um mindestens 10% dehnbar ist.

7. Verfahren zur Herstellung eines alloplastischen Materials nach einem der Ansprüche 1 bis 6, dadurch gekennzeichnet, dass zur Bildung je einer Faser (5) dienende Drähte in eine aus einem anderen metallischen Material bestehende Matrix eingebettet und zusammen mit dieser durch Verformen länger sowie dünner gemacht werden und dass danach die Matrix mit einer Säure herausgelöst und dadurch ein Bündel (3) von Fasern (5) gebildet wird.

8. Verfahren nach Anspruch 7, dadurch gekennzeichnet, dass die Matrix aus einem Material gebildet wird, das weicher als das die Drähte bzw. Fasern (5) bildende metallische Material ist und vorzugsweise in Bezug auf Verlängerungen mindestens die gleiche Duktilität hat wie das die Drähte bzw. Fasern (5) bildende Material, wobei die Matrix zum Beispiel aus Kupfer oder einer Kupfer-Nickel-Legierung besteht.

9. Verfahren nach Anspruch 7 oder 8, dadurch gekennzeichnet, dass zumindest der Schlussteil der zur Bildung von Fasern (5) durch Verlängern und Querschnittsverminderung vorgenommenen Verformung eine Kaltverformung ist, wobei der aus den Drähten und der Matrix gebildete Verbund-Rohling zum Beispiel durch Pressen und/oder Walzen und/oder Ziehen verformt wird.

10. Verfahren nach einem der Ansprüche 7 bis 9, dadurch gekennzeichnet, dass die Fasern (5), nach ihrer Bildung durch eine verlängernde Querschnittsflächenverminderung von Drähten, einer Wärmebehandlung, wie Glühen, zum Beispiel Weichglühen oder Lösungsglühen, und/oder Auslagern, unterzogen werden.

Fig. 1

1

5

D

3

θ

5

d

a

3

Fig. 2

# Fig. 1

1

5

D

3

Θ

# Fig. 2

5

d

3

a

# Fig. 3

# Fig. 4

# Fig. 5